# EUROPEAN PATENT APPLICATION

(11) **EP 3 425 604 A1**
(43) Date of publication of application: **09.01.2019**
(21) Application number: 16871747.8
(22) Date of filing: 24.11.2016
(51) Int. Cl.: G08C 17/02

(54) **ELECTRONIC DEVICE, ELECTRONIC DEVICE SYSTEM, INFORMATION PROCESSING METHOD, AND INFORMATION PROMPTING METHOD**

(30) Priority: 02.03.2016 CN 201610118490
(71) Applicant: BOE Technology Group Co., Ltd., Beijing 100015 (CN)
(72) Inventor: HU, Nannan, Beijing 100176 (CN)
(74) Representative: Brötz, Helmut
(86) International application number: PCT/CN2016/107057
(87) International publication number: WO 2017/148185

(57) **Abstract**

An electronic apparatus (100) is capable of communicating with another apparatus, the electronic apparatus (100) includes: an input device (110), configured to receive input of a user operating body; a detecting device (120), configured to detect a user's physiological sign information based on the input of the user operating body to obtain a detection result; and a transmitting device (130), configured to transmit the detection result to the another apparatus so as to provide the detection result to the user through the another apparatus. The electronic apparatus (100) allows a user to conveniently learn his/her own health condition in daily life, which facilitates use of the user, and enhances user experience. Also provided are an electronic apparatus system, an information processing method and an information prompt method.

## Description

### TECHNICAL FIELD

At least one embodiment of the present disclosure relates to an electronic apparatus, an electronic apparatus system, an information processing method, and an information prompt method.

### BACKGROUND

At present, non-invasive medical detecting products have been widely used by users; the product, by means of contacting a measuring instrument with skin of a measured object, etc., indirectly guides or senses a physiological or biochemical parameter related to a life body.

Currently, there are more wearable devices for detecting respective physiological indexes of a human body appearing on a market, and these wearable devices can detect the physiological indexes of the human body, but need a mobile terminal to download an application before they can be used, and their cost is higher.

### SUMMARY

At leas one embodiment of the present disclosure provides an electronic apparatus, capable of communicating with another apparatus, the electronic apparatus comprising: an input device, configured to receive input of a user operating body; a detecting device, configured to detect a user's physiological sign information based on the input of the user operating body to obtain a detection result; and a transmitting device, configured to transmit the detection result to the another apparatus so as to provide the detection result to the user through the another apparatus.

For example, the detecting device comprises: a detection light source, a light-receiving unit and a processing unit, the detection light source is configured to emit light toward the user operating body; the light-receiving unit is configured to receive light reflected from the user operating body; and the processing unit is configured to process the light reflected back so as to obtain the user's physiological sign information.

For example, the processing unit comprises a photoelectric conversion unit and a digital signal processing unit, the photoelectric conversion unit is configured to convert an optical signal of light received by the light-receiving unit into an electrical signal; and the digital signal processing unit is configured to calculate the user's physiological sign information according to the electrical signal.

For example, the input device is a key of the electronic apparatus.

For example, the electronic apparatus is a remote control for controlling the another apparatus, and the key is a power key of the remote control.

For example, the input of the user operating body received by the input device comprises a pressing operation performed on the input device.

For example, the user operating body comprises a finger of the user.

For example, a portion of the key in contact with the user operating body is made of a transparent material, and the detecting device is located below the transparent material.

For example, the input device is configured to, after receiving the input signal, transmit an ON instruction to the detection light source, so as to turn on the detection light source.

For example, the physiological sign information comprises at least one of blood glucose detection and blood oxygen content.

At leas one embodiment of the present disclosure provides an electronic apparatus system, comprising a first electronic apparatus and at least one second electronic apparatus, the first electronic apparatus being capable of communicating with the at least one second electronic apparatus through a communication medium. The first electronic apparatus comprising: an input device, configured to receive input of a user operating body; a detecting device, configured to detect a user's physiological sign information based on the input of the user operating body; a transmitting device, configured to transmit detection result data to the second electronic apparatus. Each second electronic apparatus comprising: a receiving device, configured to receive the detection result data; and a prompting device, configured to prompt the user of the detection result data.

For example, the prompting device comprises: at least one of a display unit and a loudspeaker, for displaying or voice broadcasting the detection result data for the user.

For example, the second electronic apparatus further comprises a data processing device, which is configured for converting the detection result data received by the receiving device into at least one of image data or audio data, and then transmitting to the prompting device.

For example, the communication medium comprises: an infrared transmission medium, a Bluetooth transmission medium, or a near field communication transmission medium.

For example, the second electronic apparatus is a smart television, and the first electronic apparatus is a remote control of the smart television.

At leas one embodiment of the present disclosure provides an information processing method, adaptable in an electronic apparatus, the electronic apparatus being capable of communicating with another electronic apparatus, and the method comprising: receiving input of a user operating body; detecting a user's physiological sign information based on the input of the user operating body; and transmitting detection result data to the another electronic apparatus, so as to prompt the user of the detection result data through the other electronic apparatus.

For example, detecting of a user's physiological sign information based on the input of the user operating body comprises: emitting detection light toward the user operating body; receiving light reflected from the user operating body; and processing the light reflected back so as to obtain the user's physiological sign information.

For example, processing of the light reflected back so as to obtain the user's physiological sign information comprises: converting the light reflected back into an electrical signal; and calculating the user's physiological sign information based on the electrical signal.

For example, receiving of input of a user operating body comprises: receiving the input of the user operating body to a key of the electronic apparatus.

For example, the electronic apparatus is a remote controller for controlling the another electronic apparatus, and receiving of the input of the user operating body to a key of the electronic apparatus comprises: receiving the input of the user operating body to a power key of the remote control.

For example, receiving of input of a user operating body comprises: receiving press input of the user operating body to the electronic apparatus.

For example, after receiving input of a user operating body, the method further comprises: transmitting an ON instruction for turning on the detection light source according to the input signal, so as to turn on the detection light source to emit detection light toward the user operating body.

For example, the physiological sign information comprises at least one of blood glucose detection and blood oxygen content.

At leas one embodiment of the present disclosure provides an information prompt method, the method comprising: receiving, by a first electronic apparatus, input of a user operating body; detecting, by the first electronic apparatus, user's physiological sign information based on the input of the user operating body; transmitting, by a first electronic apparatus, detection result data to a second electronic apparatus connected therewith through a communication medium; receiving, by the second electronic apparatus, the detection result data; and prompting, by the second electronic apparatus, the user of the detection result data.

For example, prompting, by the second electronic apparatus, the user of the detection result data comprises: displaying or voice broadcasting the detection result data for the user.

For example, prompting, by the second electronic apparatus, the user of the detection result data comprises: converting, by the second electronic apparatus, the received detection result data into image data or audio data, to prompt the user of the detection result data.

For example, the second electronic apparatus is a smart television, and the first electronic apparatus is a remote control of the smart television.

### BRIEF DESCRIPTION OF THE DRAWINGS

In order to clearly illustrate the technical solution of the embodiments of the invention, the drawings of the embodiments will be briefly described in the following; it is obvious that the described drawings are only related to some embodiments of the invention and thus are not limitative of the invention.
FIG. 1a is a structural schematic diagram of an electronic apparatus according to an embodiment of the present disclosure;
FIG. 1b is a structural schematic diagram of respective parts in the electronic apparatus according to the embodiment of the present disclosure;
FIG. 2 is a flow chart of an information processing method according to the present disclosure;
FIG. 3 is a structural schematic diagram of an electronic apparatus system according to the present disclosure;
FIG. 4 is a flow chart of an information prompt method according to the present disclosure.

Reference signs:
100 - electronic apparatus; 110 - input device; 111 - contact portion; 112 - edge portion; 120 - detecting device; 121 - detection light source; 122 - light-receiving unit; 123 - processing unit; 1231 - photoelectric conversion unit; 1232 - digital signal processing unit; 130 - transmitting device; 310 - first electronic apparatus; 311 - input device; 312 - detecting device; 313 - transmitting device; 320 - second electronic apparatus; 321 - receiving device; 322 - prompting device; 323 - data processing device.

### DETAILED DESCRIPTION

In order to make objects, technical details and advantages of the embodiments of the invention apparent, the technical solutions of the embodiments will be described in a clearly and fully understandable way in connection with the drawings related to the embodiments of the invention. Apparently, the described embodiments are just a part but not all of the embodiments of the invention. Based on the described embodiments herein, those skilled in the art can obtain other embodiment(s), without any inventive work, which should be within the scope of the invention.

Unless otherwise defined, all the technical and scientific terms used herein have the same meanings as commonly understood by one of ordinary skill in the art to which the present invention belongs. The terms "first," "second," etc., which are used in the description and the claims of the present application for invention, are not intended to indicate any sequence, amount or importance, but distinguish various components. Also, the terms such as "a," "an," etc., are not intended to limit the amount, but indicate the existence of at least one. The terms "comprise," "comprising," "include," "including," etc., are intended to specify that the elements or the objects stated before these terms encompass the elements or the objects and equivalents thereof listed after these terms, but do not preclude the other elements or objects. The phrases "connect", "connected", etc., are not intended to define a physical connection or mechanical connection, but may include an electrical connection, directly or indirectly. "On," "under," "right," "left" and the like are only used to indicate relative position relationship, and when the position of the object which is described is changed, the relative position relationship may be changed accordingly.

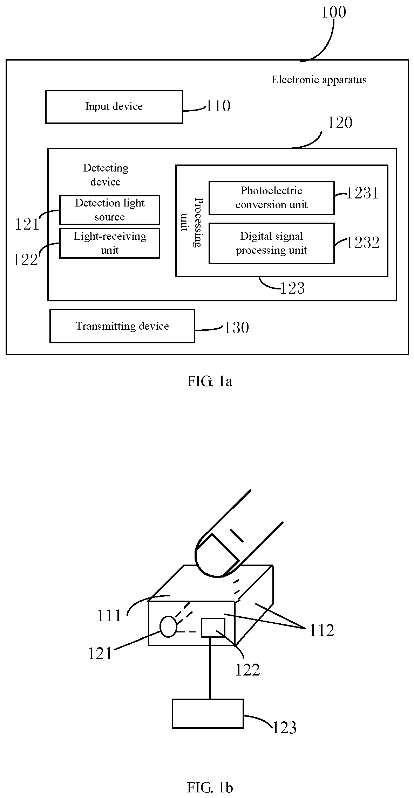

FIG. 1a shows a structural diagram of an electronic apparatus according to a first embodiment of the present disclosure. FIG. 1b is a structural schematic diagram of respective parts in the electronic apparatus according to the embodiment of the present disclosure. Hereinafter, respective parts in the electronic apparatus and positional structural relationship of the respective parts will be described in conjunction with FIG. 1a and FIG. 1b. The electronic apparatus 100 may be an apparatus capable of communicating with another apparatus. For example, the electronic apparatus includes, but is not limited to, a computer mouse, a computer keyboard, a television remote control, a telephone, a mobile terminal, a digital camera, a tablet personal computer, a laptop and a game console, and so on. With reference to FIG. 1a, the electronic apparatus 100 may be connected with another electronic apparatus through a communication medium, for example, by wired means or wireless means. The communication medium of the communication by the wired means may be: for example, an electrical data line, an optical fiber, and a telephone line; and the wireless communication means may be: for example, infrared communication, Bluetooth communication, wireless fidelity (WIFI) communication, etc.

With reference to FIG. 1a, the electronic apparatus 100 comprises at least: an input device 110, a detecting device 120, and a transmitting device 130. The input device 110 is in signal connection with the detecting device 120, and the detecting device 120 is in signal connection with the transmitting device 130. The input device 110 is configured to receive input of a user operating body. The detecting device 120 is configured to detect a user's physiological sign information based on the input of the user operating body, to obtain a detection result. The transmitting device 130 transmits detection result data to other apparatus, so as to provide the detection result to the user through the another apparatus.

The user operating body may be a body part when the user operates the electronic apparatus, for example, when the user operates with a hand, the user operating body may be a specific part of the hand or the entire hand.

The input device 110 is used for receiving the input of the user operating body. According to an example of the present disclosure, the input device 110 may be a key of the electronic apparatus 100, e.g., a keyboard, a mouse, a remote control, a touch screen, and the like; for example, a keyboard key, a mouse button, a remote control button, an on-offkey or a touch panel of a touch screen, and so on. With reference to FIG. 1b, the input device 110 of the electronic apparatus 100 includes a contact portion 111 and an edge portion 112; the contact portion 111 is used for contacting with the user operating body, and the portion may be made of a transparent material with light transmittance. The edge portion 112 may be a support portion of a key, and the portion may be made of a non-transparent material without light transmittance. The detecting device 120 is located below the contact portion 111, and due to light transmittance of the contact portion and non-light transmittance of the edge portion, the detecting device 120 can obtain only information of the contact portion between the user operating body and the input device 110, to avoid interference of other information, so as to improve detection accuracy. Optionally, the key is a power key of the electronic apparatus, for example, a power key of the television remote control. The user usually presses the power key every day to turn on the television, in this way, the input device is provided in the power key, when the user turns on the television every day, he/she may have his/her physiological sign information detected, without deliberately taking out a medical device for detecting as in the prior art, which greatly facilitates use of the user. According to an example of the present disclosure, the user operating body may be, for example, a finger, a palm, etc. of the user. The input device 110 may receive operation of the user operating body; for example, receive a pressing operation, a holding operation, a touch operation, etc. of the operating body. The input device 110, after receiving the above-described operation of the user, generates an input signal, and the electronic apparatus 100, after receiving the input signal, may transmit an ON instruction to the detecting device 120 of the electronic apparatus 100, to instruct the detecting device 120 to detect the operating body.

The detecting device 120 is configured to detect the user's physiological sign information based on the user's input. According to an example of the present disclosure, the detecting device 120 may include: a detection light source 121, a light-receiving unit 122, and a processing unit 123.

The detection light source 121 is configured to emit detection light toward the user operating body. With reference to FIG. 1b, the detection light source 121 may be provided below the input device 110, to emit detection light to the user operating body when the user presses the input device. The detection light may be, for example, laser, infrared light, or near infrared light, etc. The detection light source may be implemented by, for example, a laser, an infrared generator, etc. Optionally, it may also be implemented by a near infrared tunable fiber laser, an infrared emission tube, or the like. The infrared emission tube consists of an infrared light emitting diode matrix. An infrared emission diode is made of a material with high infrared radiation efficiency into a PN junction, and forward bias is applied to inject the PN junction with current for excitation of infrared light. The infrared light emitting diode is usually made of gallium arsenide (GaAs), arsenic gallium arsenide (GaAlAs) and other materials, and encapsulated with a fully transparent or light blue or black resin.

The light-receiving unit 122 is configured to receive light reflected from the user operating body. The light-receiving unit 122 may receive light that is not absorbed by a human body but reflected by the operating body, and transmit the light into the processing unit 123, or pre-process the same before transmitting into the processing unit 123. In one example, the light-receiving unit 122 may be implemented by, for example, an optical receiver. The optical receiver includes a receiving antenna, which may collect spatially propagated light field and converge the same onto a detector surface. Thereby, with minimal additional noise and distortion, information carried by a light carrier is recovered. In addition, the optical receiver is also capable of performing amplifying, reshaping, and regenerating a transmitted signal on an optical signal.

The processing unit 123 is configured to process light transmitted by the light-receiving unit 122 and reflected by the user operating body, to obtain the user's physiological sign information. Blood concentrations, hemoglobin contents, or blood glucose levels in human bodies are different, so absorption and reflection conditions of light are also different. In addition, when the user's heart is systolic, blood flow is large, so an amount of light absorption is larger; on the contrary, when the heart is diastolic, the blood flow is small, so the amount of light absorption is also small; and thus, by detecting regular variation of the amount of light absorption, a heartbeat condition, as well as the blood concentration, the hemoglobin content, or the blood glucose level can be distinguished. Through these parameters, the user's physiological sign information, e.g., the blood glucose level, blood oxygen content, heart rate and other physiological indexes can be obtained.

The processing unit 123 is connected with the light-receiving unit 122, and obtains the physiological sign information of the human body according to intensity information of sensitive light received by the light-receiving unit 122. The processing unit 123 may include, for example, a photoelectric conversion unit 1231 and a digital signal processing unit 1232. The photoelectric conversion unit 1231 is configured to convert light received by the light-receiving unit 122 into an electrical signal; and the digital signal processing unit 1232 is configured to calculate the user's physiological sign information according to the electrical signal. The processing unit 123 may, for example, be implemented by a microprocessor chip, and perform receiving, analyzing and processing a data signal. Optionally, the processing unit 123 may also filter out noise or interference from the optical signal received by the light-receiving unit 122. In addition, the processing unit 123 may also include a memory, a sensor, etc., according to needs, for implementing storage, detection, and the like of the signal.

The non-invasive detection mode as described above by detecting the light absorption condition of the operating body is only an example of the present disclosure, and it can be understood by those skilled in the art that the detecting device 120 may further be other forms of detecting devices. For example, the detecting device 120 may be a sensor having a specific function; for example, a biosensor, a capacitive sensor, or the like. A sensor signal outputting part of a sensor is provided thereon with a positive electrode, and a sensor signal receiving part is provided thereon with a negative electrode; when the sensor signal outputting part and the sensor signal receiving part are respectively in contact with any two different parts of the user operating body, because substances (for example, enzymes, cells, proteins, microorganisms, etc.) contained by body tissue and body fluid of the human body can all be conductive, a sensor drive signal transmitted by the sensor signal outputting part can be transmitted to the sensor signal receiving part through the user operating body or secretions of the operating body. At this time, the positive electrode and the negative electrode are conducted, and the sensor can start performing a sensing operation. Dielectric constants of the body tissues or the body fluids of different users with respect to current conduction are different, and therefore, the user's physiological sign information can be learned by detecting the dielectric constant. When the detecting device 120 is the capacitive sensor, the dielectric constant of the user operating body can be detected according to a change value of capacitance in a circuit. When the detecting device 120 is the biosensor, it is possible to scan an electrocardiographic change image by an internal electrocardiographic scanner thereof, so as to obtain information related to electrocardiographic change, such as a pulse, of the user operating body.

When the detecting device 120 detects the user's physiological sign information based on the user operating body, the transmitting device 130 transmits the detection result data to other electronic apparatus, so as to prompt the user of the detection result data through the apparatus. For example, after the computer mouse, as the electronic apparatus 100, detects the user's physiological sign information, it transmits the same to other electronic apparatus connected therewith, for example, a host computer, and prompts the user of the detection result through an output device of the host computer. After the tablet personal computer, as the electronic apparatus 100, detects the user's physiological sign information, it transmits the same to other electronic apparatus connected therewith, for example, the mobile terminal, and prompts the user of the detection result through an output device of the mobile terminal. According to an example of the present disclosure, the other electronic apparatus has a display function, and the electronic apparatus 100 may transmit the detection result data to the other electronic apparatus, so as to display the detection data through the apparatus. Alternatively, the other electronic apparatus has a loudspeaker, and the electronic apparatus 100 may also transmit the detection result data to the apparatus, so as to perform voice broadcast on the detection data through the other electronic apparatus, to facilitate the user to learn his/her health condition. For example, when the electronic apparatus 100 is a television remote control, and when the user wants to watch the television daily, he/she presses the power key of the television, and the television remote control can detect the user's blood glucose content information through the user's press, and at the same time, when the user presses the power key, the television is turned on, and the blood glucose content information may be displayed through the display screen of a display, and broadcast through the loudspeaker of the television. The user learns the above-described information of his/her own, and then enters a television program screen.

With the above-described embodiment provided by the present disclosure, since the user usually turns on the television daily with the remote control, for example, turns on the television by pressing the power key, when the user turns on the television every day, he/she may have his/her physiological sign information detected, without deliberately purchasing and using the medical device for detecting as in the conventional technology, which greatly facilitates the use of the user.

The electronic apparatus according to the first embodiment of the present disclosure is described above, and an information processing method according to a second embodiment of the present disclosure will be further described below. The information processing method corresponds to the electronic apparatus according to the first embodiment, and for conciseness of the description, it will only be described concisely below.

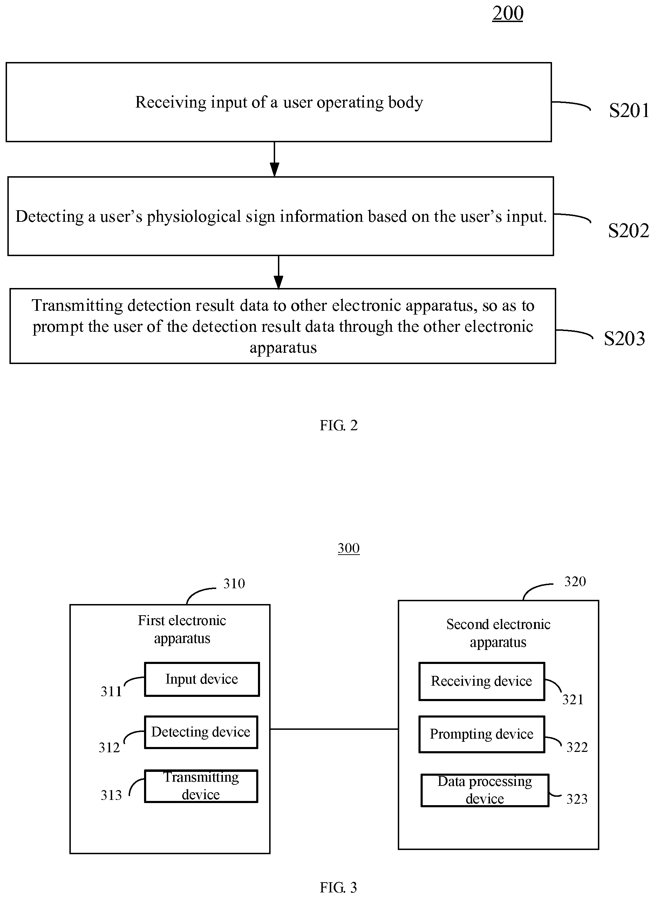

FIG. 2 shows a flow chart of the information processing method according to the second embodiment of the present disclosure, and the information processing method 200 is used in the electronic apparatus 100 shown in FIG. 1. The information processing method according to the second embodiment of the present disclosure will be described below with reference to FIG. 1 and FIG. 2. The electronic apparatus 100 includes, but is not limited to, a computer mouse, a computer keyboard, a television remote control, a telephone, a mobile terminal, a digital camera, a tablet personal computer, a laptop and a game console, and so on. The electronic apparatus may be connected with other electronic apparatus through a communication medium, for example, by wired means or wireless means. The communication medium of the communication by the wired means may be, for example, an electrical line, an optical fiber, or a telephone line; and the wireless communication means may be, for example, infrared communication, Bluetooth communication, wireless fidelity (WIFI) communication, etc.

With reference to FIG. 2, in step S201, receiving input of a user operating body. According to an example of the present disclosure, an input device 110 of the electronic apparatus 100 is used for receiving the input of the user operating body. For example, a keyboard key, a mouse button, a remote control button, an on-off key or a touch panel of a touch screen and so on are used. Optionally, the key is a power key of the electronic apparatus, for example, a power key of a television remote control. In this way, the user does not have to deliberately take out a medical device for detecting, instead, when he/she turns on the television every day, he/she may have his/her physiological sign information detected, which greatly facilitates use of the user. According to an example of the present disclosure, the user operating body may be, for example, a finger, a palm, etc. of the user. For example, the receiving input of a user operating body may include receiving a pressing operation, a holding operation, a touch operation, and the like. The input device, after receiving the above-described input of the user, generates an input signal, and based on the input signal, the electronic apparatus 100 may transmit an ON instruction to the detecting device, to instruct the detecting device to detect the operating body.

In step S202, detecting a user's physiological sign information based on the user's input. According to an example of the present disclosure, the user's physiological sign information is detected by using the detecting device 120 in the electronic apparatus 100. For a specific composition of the detecting device 120 and a specific function of each part thereof, the electronic apparatus according to the first embodiment may be referred to. It will not be repeated here.

For example, detection light is firstly emitted toward the user operating body. The detection light may be, for example, laser, infrared light, or near infrared light, etc. Then, light reflected from the user operating body is received. After the reception, the light is processed to obtain the user's physiological sign information. Blood concentrations, hemoglobin contents, or blood glucose levels in human bodies are different, so absorption and reflection conditions of light are also different. In addition, when the user's heart is systolic, blood flow is large, so an amount of light absorption is larger; on the contrary, when the heart is diastolic, the blood flow is small, so the amount of light absorption is also small; and thus, by detecting regular variation of the amount of light absorption, a heartbeat condition, as well as the blood concentration, the hemoglobin content, or the blood glucose level can be distinguished. Through these parameters, the user's physiological sign information, e.g., the blood glucose level, blood oxygen content, heart rate and other physiological indexes can be obtained.

According to an example of the present disclosure, the light reflected from the user operating body may be firstly converted into an electrical signal, and the user's physiological sign information is further calculated based on the electrical signal.

The non-invasive detection mode as described above by detecting the light absorption condition of the operating body is only an example of the present disclosure, and it can be understood by those skilled in the art that other detection methods may be used, for example, by detecting the physiological sign information of the operating body with a sensor having a specific function; for example, a biosensor, a capacitive sensor, and the like. A sensor signal outputting part of a sensor is provided thereon with a positive electrode, and a sensor signal receiving part is provided thereon with a negative electrode; when the sensor signal outputting part and the sensor signal receiving part are respectively in contact with any two different parts of the user operating body, since substances (for example, enzymes, cells, proteins, microorganisms, etc.) contained by body tissue and body fluid of the human body can all be conductive, a sensor drive signal transmitted by the sensor signal outputting part can be transmitted to the sensor signal receiving part through the user operating body or secretions of the operating body. At this time, the positive electrode and the negative electrode are conducted, and the sensor can start performing a sensing operation; dielectric constants of the body tissues or the body fluids of different users with respect to current conduction are different, and therefore, the user's physiological sign information can be learned by detecting the dielectric constant. For example, the capacitance sensor is used for sensing a change value of capacitance in a circuit, to detect the dielectric constant of the user operating body. The biosensor is used for scanning an electrocardiographic change image with an internal electrocardiographic scanner thereof, so as to obtain information related to electrocardiographic change, such as a pulse, of the user operating body.

In step S203, transmitting detection result data to other electronic apparatus, so as to prompt the user of the detection result data through the other electronic apparatus. According to an example of the present disclosure, when in step S202, the user's physiological sign information is detected based on the user operating body, in step S203, the detection result data is transmitted to other electronic apparatus, so as to prompt the user of the detection result data through the apparatus. For example, after the user's physiological sign information is detected by using a computer mouse, it is transmitted to other electronic apparatus connected therewith, for example, a host computer, so as to prompt the user of the detection result through an output device of the host computer. After the user's physiological sign information is detected by using a tablet personal computer, it is transmitted to other electronic apparatus connected therewith, for example, the mobile terminal, so as to prompt the user of the detection result through an output device of the mobile terminal. According to an example of the present disclosure, the detection data may be displayed through other electronic apparatus. Alternatively, voice broadcast may be performed on the detection data through the other electronic apparatus, to facilitate the user to learn his/her health condition. For example, when the user wants to watch the television daily, he/she presses the power key of the television, the television remote control can detect the user's blood glucose content information through the user's press, and at the same time, when the user presses the power key, the television is turned on, and the blood glucose content information may be displayed through a display screen of a display, and broadcast through the loudspeaker of the television. After the user learns the above-described information of his/her own, it then enters a television program screen.

With the above-described embodiment provided by the present disclosure, the user can conveniently learn his/her physiological sign information and a health condition in everyday life, without deliberately purchasing and using the medical device, which greatly facilitates use of the user.

The electronic apparatus according to the first embodiment of the present disclosure and the information processing method according to the second embodiment of the present disclosure are described above. Hereinafter, an electronic apparatus system according to a third embodiment of the present disclosure will be further described, the electronic apparatus system comprising the electronic apparatus according to the first embodiment of the present disclosure and other electronic apparatus connected with the electronic apparatus. In the third embodiment, the above-described electronic apparatus is referred to as a first electronic apparatus, and the above-described other electronic apparatus is referred to as a second electronic apparatus. With respect to a structure of the first electronic apparatus, please refer to the electronic apparatus according to the first embodiment of the present disclosure, which will not be repeated here, and hereinafter, only the second electronic apparatus will be described in detail below.

FIG. 3 shows an exemplary framework diagram of the electronic apparatus system according to the third embodiment of the present disclosure. With reference to FIG. 3, the electronic apparatus system comprises: a first electronic apparatus 310, a second electronic apparatus 320, the first electronic apparatus 310 being capable of communicating with the second electronic apparatus 320 through a communication medium, for example, by wired means or wireless means. The communication medium of the communication by the wired means may be: for example, an electrical data line, an optical fiber, and a telephone line; and the wireless communication means may be: for example, infrared communication, Bluetooth communication, wireless fidelity (WIFI) communication, etc.

The first electronic apparatus 310 includes: an input device 311, configured to receive input of a user operating body; a detecting device 312, configured to detect a user's physiological sign information based on the input of the user; and a transmitting device 313, configured to transmit detection result data to the second electronic apparatus 320. For a specific solution, please refer to the electronic apparatus 100 according to the first embodiment of the present disclosure.

For example, any of the electronic apparatuses according to the first embodiment may be used as the first electronic apparatus according to this embodiment, and a specific structure and function of the electronic apparatus will not be repeated here.

In addition, the second electronic apparatus 320 includes: a receiving device 321, configured to receive the detection result data transmitted from the first electronic apparatus 320. The receiving device 321, after receiving the detection result data, transmits the data to the prompting device 322 to prompt the user of the information. The receiving device 321 may be, for example, an infrared receiver, an optical receiver, a central processing unit, etc. The prompting device 322 is configured to prompt the user of the detection result data. The prompting device 322 may be an output device of the electronic apparatus, for example, a display unit or a loudspeaker. The display unit may display the detection result data to the user; and the loudspeaker may perform voice broadcast on the detection result data for the user.

In addition, according to an example of the present disclosure, the second electronic apparatus 320 may further include a data processing device 323, the data processing device 323 being capable of converting the detection result data received by the receiving device 321 into image data or audio data, and then transmitting to the prompting device 322.

According to an example of the present disclosure, the first electronic apparatus 310 is a television remote control and the second electronic apparatus 320 is a smart television. When the user wants to watch television, he/she presses the power key of the television remote control, and the television remote control can detect the user's blood glucose content information through the user's press. At the same time, when the user presses the power key, the smart television is turned on, and the blood glucose content information may be displayed through a display screen of the television, and broadcast through the loudspeaker of the television. After a predetermined time for finishing display of the above-described information, or, according to confirm option of the user, the smart television then enters the television program screen. Alternatively, a detection result prompt screen may also replace a boot screen, which will not affect normal viewing of the television program.

With the electronic apparatus system provided by the embodiment of the present disclosure, the user does not need to purchase the medical device, instead, he/she learns his/her own health condition during daily use of a household appliance, which expands a function of the household appliance, saves a fabrication material, and facilitates use of the user.

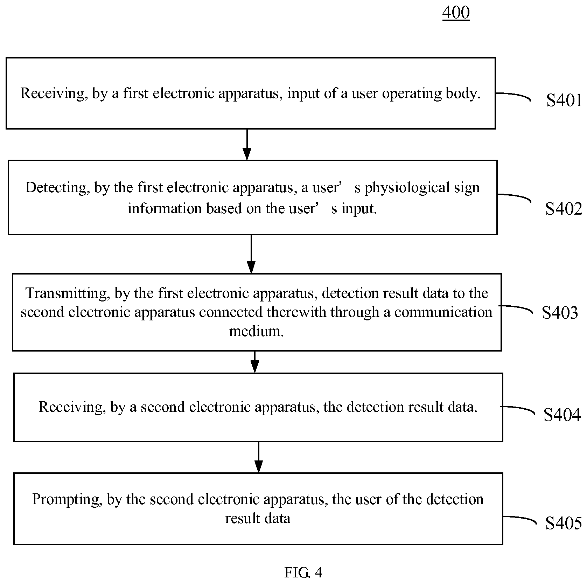

The electronic apparatus system according to the third embodiment of the present disclosure is described above, and an information prompt method according to a fourth embodiment of the present disclosure will be further described below. FIG. 4 is a flow chart of the information prompt method according to the fourth embodiment of the present disclosure. FIG. 3 is a structural schematic diagram of the electronic apparatus system according to the third embodiment of the present disclosure. The information prompt method according to the fourth embodiment corresponds to the electronic apparatus system according to the third embodiment. The information prompt method according to the fourth embodiment will be described below with reference to FIG. 3 and FIG. 4.

With reference to FIG. 4, in step S401, receiving, by the first electronic apparatus, input of a user operating body. In step S402, detecting, by the first electronic apparatus, user's physiological sign information based on the user's input. In step S403, transmitting, by the first electronic apparatus, detection result data to the second electronic apparatus connected therewith through a communication medium. The above-described three steps are the same as those of the information processing method applied to the electronic apparatus according to the second embodiment of the present disclosure, which will not be repeated here.

In step S404: receiving, by the second electronic apparatus, the detection result data. According to an example of the present disclosure, a receiving device 321 of the second electronic apparatus receives the detection result data from the first electronic apparatus and, after receiving the detection result data, transmits the data to a prompting device 322 to prompt the user of the information. The receiving device 321 may be, for example, an infrared receiver, an optical receiver, a central processing unit, etc.

In step S405: prompting, by the second electronic apparatus, the user of the detection result data. According to an example of the present disclosure, the second electronic apparatus prompts the use of the detection result data by using the prompting device 322. The prompting device 322 may be an output device of the electronic apparatus, for example, a display unit or a loudspeaker. The display unit may display the detection result data to the user; and the loudspeaker may perform voice broadcast on the detection result data for the user.

In addition, according to an example of the present disclosure, the second electronic apparatus 320 may convert the detection result data received by the receiving device 321 into image data or audio data, by using a data processing device 323, and then transmit to the prompting device 322.

According to an example of the present disclosure, the first electronic apparatus 310 is a television remote control and the second electronic apparatus 320 is a smart television. When the user wants to watch television, he/she presses the power key of the television remote control, and the television remote control can detect the user's blood glucose content information through the user's press. At the same time, when the user presses the power key, the smart television is turned on, and the blood glucose content information may be displayed through a display screen of the television, and broadcast through the loudspeaker of the television. After a predetermined time for finishing display of the above-described information, or, according to confirm option of the user, the smart television then enters the television program screen.

By using the information prompt method according to the embodiment of the present disclosure, the user does not need to purchase the medical device, instead, he/she learns his/her own health condition during daily use of a household appliance, which enables the household appliance more intellectualized, and facilitates use of the user.

The above merely is specific embodiments of the present disclosure, and not intended to define the scope of the present disclosure. Any variations or replacements which can be easily thought of by those skilled in the art in the scope of the present disclosure all shall fall within the scope of protection of the present disclosure. Therefore, the scope of the present disclosure should be the scope of the following claims.

The present application claims priority of Chinese Patent Application No. 201610118490.4 filed on March 2, 2016, the present disclosure of which is incorporated herein by reference in its entirety as part of the present application.

## Claims

1. An electronic apparatus, capable of communicating with another apparatus, the electronic apparatus comprising:
an input device, configured to receive input of a user operating body;
a detecting device, configured to detect a user's physiological sign information based on the input of the user operating body to obtain a detection result; and
a transmitting device, configured to transmit the detection result to the another apparatus so as to provide the detection result to the user through the another apparatus.

2. The electronic apparatus according to claim 1, wherein the detecting device comprises:
a detection light source, a light-receiving unit and a processing unit,
the detection light source is configured to emit light toward the user operating body;
the light-receiving unit is configured to receive light reflected from the user operating body; and
the processing unit is configured to process the light reflected back so as to obtain the user's physiological sign information.

3. The electronic apparatus according to claim 2, wherein the processing unit comprises a photoelectric conversion unit and a digital signal processing unit,
the photoelectric conversion unit is configured to convert an optical signal of light received by the light-receiving unit into an electrical signal; and
the digital signal processing unit is configured to calculate the user's physiological sign information according to the electrical signal.

4. The electronic apparatus according to any one of claims 1 to 3, wherein the input device is a key of the electronic apparatus.

5. The electronic apparatus according to claim 4, wherein the electronic apparatus is a remote control for controlling the another apparatus, and the key is a power key of the remote control.

6. The electronic apparatus according to any one of claims 1 to 5, wherein the input of the user operating body received by the input device comprises a pressing operation performed on the input device.

7. The electronic apparatus according to any one of claims 1 to 6, wherein the user operating body comprises a finger of the user.

8. The electronic apparatus according to any one of claims 4 to 5, wherein a portion of the key in contact with the user operating body is made of a transparent material, and the detecting device is located below the transparent material.

9. The electronic apparatus according to any one of claims 2 to 8, wherein the input device is configured to, after receiving the input signal, transmit an ON instruction to the detection light source, so as to turn on the detection light source.

10. The electronic apparatus according to any one of claims 1 to 9, wherein the physiological sign information comprises at least one of blood glucose detection and blood oxygen content.

11. An electronic apparatus system, comprising a first electronic apparatus and at least one second electronic apparatus, the first electronic apparatus being capable of communicating with the at least one second electronic apparatus through a communication medium, the first electronic apparatus comprising:
an input device, configured to receive input of a user operating body;
a detecting device, configured to detect a user's physiological sign information based on the input of the user operating body; and
a transmitting device, configured to transmit detection result data to the second electronic apparatus;
each second electronic apparatus comprising:
a receiving device, configured to receive the detection result data; and
a prompting device, configured to prompt the user of the detection result data.

12. The electronic apparatus system according to claim 11, wherein the prompting device comprises: at least one of a display unit and a loudspeaker, for displaying or voice broadcasting the detection result data for the user.

13. The electronic apparatus system according to any one of claims 11 to 12, wherein the second electronic apparatus further comprises a data processing device, which is configured for converting the detection result data received by the receiving device into at least one of image data or audio data, and then transmitting to the prompting device.

14. The electronic apparatus system according to any one of claims 11 to 13, wherein the communication medium comprises: an infrared transmission medium, a Bluetooth transmission medium, or a near field communication transmission medium.

15. The electronic apparatus system according to any one of claims 11 to 14, wherein the second electronic apparatus is a smart television, and the first electronic apparatus is a remote control of the smart television.

16. An information processing method, adaptable in an electronic apparatus, the electronic apparatus being capable of communicating with another electronic apparatus, and the method comprising:
receiving input of a user operating body;
detecting a user's physiological sign information based on the input of the user operating body; and
transmitting detection result data to the another electronic apparatus, so as to prompt the user of the detection result data through the other electronic apparatus.

17. The processing method according to claim 16, wherein detecting of a user's physiological sign information based on the input of the user operating body comprises:
emitting detection light toward the user operating body;
receiving light reflected from the user operating body; and
processing the light reflected back so as to obtain the user's physiological sign information.

18. The processing method according to claim 17, wherein processing of the light reflected back so as to obtain the user's physiological sign information comprises:
converting the light reflected back into an electrical signal; and
calculating the user's physiological sign information based on the electrical signal.

19. The processing method according to any one of claims 16 to 18, wherein receiving of input of a user operating body comprises:
receiving the input of the user operating body to a key of the electronic apparatus.

20. The processing method according to claim 19, wherein the electronic apparatus is a remote controller for controlling the another electronic apparatus, and receiving of the input of the user operating body to a key of the electronic apparatus comprises:
receiving the input of the user operating body to a power key of the remote control.

21. The processing method according to claim 16, wherein receiving of input of a user operating body comprises:
receiving press input of the user operating body to the electronic apparatus.

22. The processing method according to claim 17, wherein after receiving input of a user operating body, the method further comprises:
transmitting an ON instruction for turning on the detection light source according to the input signal, so as to turn on the detection light source to emit detection light toward the user operating body.

23. The processing method according to any one of claims 16 to 18, wherein the physiological sign information comprises at least one of blood glucose detection and blood oxygen content.

24. An information prompt method, the method comprising:
receiving, by a first electronic apparatus, input of a user operating body;
detecting, by the first electronic apparatus, user's physiological sign information based on the input of the user operating body;
transmitting, by a first electronic apparatus, detection result data to a second electronic apparatus connected therewith through a communication medium;
receiving, by the second electronic apparatus, the detection result data; and
prompting, by the second electronic apparatus, the user of the detection result data.

25. The method according to claim 24, wherein prompting, by the second electronic apparatus, the user of the detection result data comprises:
displaying or voice broadcasting the detection result data for the user.

26. The method according to claim 24, wherein prompting, by the second electronic apparatus, the user of the detection result data comprises:
converting, by the second electronic apparatus, the received detection result data into image data or audio data, to prompt the user of the detection result data.

27. The method according to any one of claims 24 to 26, wherein the second electronic apparatus is a smart television, and the first electronic apparatus is a remote control of the smart television.
